# EUROPEAN PATENT APPLICATION

(11) **EP 4 786 585 A1**
(43) Date of publication of application: **05.08.2026**
(21) Application number: 24886332.6
(22) Date of filing: 01.11.2024
(51) Int. Cl.: C12N 9/22, C12N 15/75

(54) **SIGNAL PEPTIDE FOR PRODUCING SERRATIA MARCESCENS-DERIVED NUCLEASE AND USE THEREOF**

(30) Priority: 03.11.2023 KR 20230150911
(71) Applicant: CJ Cheiljedang Corporation, Seoul 04560 (KR)
(72) Inventor: SEOK, Jong-cheol, Seoul 04560 (KR); LEE, Joo Hee, Seoul 04560 (KR); JI, Chang Jun, Seoul 04560 (KR); CHO, A-Ra, Seoul 04560 (KR); LEE, Hyo Hyoung, Seoul 04560 (KR)
(74) Representative: Meissner Bolte Partnerschaft mbB
(86) International application number: PCT/KR2024/017017
(87) International publication number: WO 2025/095661

(57) **Abstract**

The present disclosure relates to a signal peptide for producing a *Serratia* marcescens-derived nuclease and a use thereof.

## Description

### [Technical Field]

The present disclosure relates to a signal peptide for increasing *Serratia marcescens*-derived nuclease-producing ability and a method for producing a nuclease using the same.

### [Background Art]

Nucleases are enzymes that cleave phosphodiester bonds in nucleic acids, and are enzymes that perform important roles in the replication, repair, recombination, and degradation of DNA and RNA in living organisms. In particular, non-specific nucleases are used for pharmaceutical and research purposes in viral infection, cancer diagnosis, production of gene therapeutics and vaccines, and the like, and are also widely used for research in the field of molecular biology. In addition, such nucleases are used for food and industrial purposes, such as the removal of residual nucleic acids in fermented foods, improvement in viscosity of fermentation broths, and removal of biofilms, and thus are enzymes with very high industrial values.

Although various microorganisms produce nucleases, among them, nucleases derived from the Gram-negative bacterium *Serratia marcescens* are known to have non-specific properties of cleaving all forms of DNA and RNA, including single-stranded, double-stranded, linear, and circular forms, and to exhibit high specific activity. Accordingly, many studies have been conducted to produce *Serratia marcescens*-derived nucleases (US 9,796,994 B2).

However, when a nuclease is overexpressed and exists in an active state within the cytoplasm of a host cell, it may also degrade nucleic acids in the host and thereby inhibit cell growth. Thus, there are difficulties in producing nucleases using microorganisms. To date, cases of producing up to 5,000 U/mL of nuclease using such a method have been reported, and commercially available products produced using this method are still very expensive. Therefore, it is important to select an optimal secretion signal peptide for nuclease production.

Meanwhile, *Bacillus subtilis* is considered a GRAS (Generally Regarded As Safe) organism,, and has excellent capacity for protein secretion, and thus has been used as a host strain for the production of various recombinant proteins. In particular, studies on secretion signal peptides for protein secretion have been conducted. To date, various screening techniques and optimization methods for the production of foreign proteins have been studied; however, optimal signal peptides for the secretory production of *Serratia marcescens*-derived nucleases have rarely been studied (He Li et al., Biochemical Engineering Journal, 189 (2022) 108718).

### [Disclosure]

### [Technical Problem]

The present inventors have confirmed that *Serratia marcescens*-derived nuclease-producing ability can be increased by using a signal peptide, thereby completing the present disclosure.

### [Technical Solution]

An object of the present disclosure is to provide a polypeptide comprising: the amino acid sequence of SEQ ID NO: 5 or 7; and an amino acid sequence of a *Serratia marcescens*-derived nuclease.

Another object of the present disclosure is to provide a polynucleotide encoding the polypeptide.

Still another object of the present disclosure is to provide a microorganism comprising the polypeptide or a polynucleotide encoding the same.

Still another object of the present disclosure is to provide a method for producing a *Serratia marcescens*-derived nuclease, comprising: culturing a microorganism, comprising the polypeptide or a polynucleotide encoding the same, in a medium; and recovering a nuclease from the culture.

Still another object of the present disclosure is to provide a composition for producing a *Serratia marcescens*-derived nuclease, comprising a signal peptide having the amino acid sequence of SEQ ID NO: 5 or 7.

### [Advantageous Effects]

The present disclosure provides a signal peptide for increasing *Serratia marcescens*-derived nuclease-producing ability, and thus can be used for the mass production of a *Serratia marcescens*-derived nuclease.

### [Brief Description of the Drawings]

FIG. 1 is a diagram illustrating the respective halo sizes generated as a result of screening nuclease-producing strains for each signal peptide.
FIG. 2 is a diagram illustrating the results of confirming the nuclease expression level over time in strains cultured using a 5 L fermenter.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below. Furthermore, those skilled in the art may be able to recognize or identify numerous equivalents to the particular aspects of the present disclosure described herein by using routine experimentation. Moreover, these equivalents are intended to be included in the present disclosure.

The terms used herein are as described below.

### Proteins, Polypeptides

As used herein, the term "protein" or "polypeptide" refers to a polymer or oligomer of consecutive amino acid residues. In the present disclosure, "polypeptide", "protein", and "peptide" may be used interchangeably.

In the present disclosure, unless indicated otherwise, amino acid sequences are described in the N-terminal to C-terminal direction.

In the present disclosure, with respect to amino acid sequences, it is apparent that a polypeptide or protein "comprising" an amino acid sequence set forth in a specific sequence number, a polypeptide or protein "consisting of" an amino acid sequence set forth in a specific sequence number, or a polypeptide or protein "having" an amino acid sequence set forth in a specific sequence number may also comprise a polypeptide or protein in which specific amino acid(s) are deleted, modified, substituted, or added, as long as it has the same or corresponding activity as the polypeptide or protein consisting of the amino acid sequence of the sequence number. For example, the polypeptide or protein may also comprise, in the internal region or the region upstream or downstream (N-terminus or C-terminus) of the polypeptide or protein, a polypeptide or protein having addition or deletion of amino acid(s), naturally-occurring mutations, silent mutations, or conservative substitutions that do not alter the functions of the protein of the present disclosure, as long as the polypeptide or protein exhibits the same or corresponding activity.

### Polynucleotides

As used herein, the term "polynucleotide", "nucleic acid", or "nucleic acid molecule" refers to a DNA (*e.g.*, cDNA or genomic DNA) or RNA (*e.g.*, mRNA) strand having at least a certain length, which is a polymer of nucleotides in which nucleotide monomers are connected in a long chain by covalent bonds. In the present disclosure, "polynucleotide", "nucleic acid", and "nucleic acid molecule" may be used interchangeably.

### Identity, Homology

As used herein, the term "identity" or "homology" refers to the degree of similarity between two given amino acid or base sequences and may be expressed as a percentage. In the present disclosure, the terms "homology" and "identity" may often be used interchangeably.

The sequence homology or identity of conserved polynucleotides or polypeptides can be determined by a standard alignment algorithm, and a default gap penalty established by the program used may be applied together.

Whether any two polynucleotide or polypeptide sequences have homology, similarity, or identity may be determined by, for example, a known computer algorithm such as the "FASTA" program using default parameters as described in Pearson et al. (1988) [Proc. Natl. Acad. Sci. USA 85]: 2444. Alternatively, it may be determined by comparing the sequence information using the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later), or using a GAP computer program such as the Smith-Waterman algorithm (Smith and Waterman, Adv. Appl. Math (1981) 2:482) (the GCG program packages (Devereux, J. et al., Nucleic Acids Research 12:387 (1984)), BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are included). For example, the homology, similarity, or identity may be determined by using BLAST of the National Center for Biotechnology Information or ClustalW.

Further, whether any two polynucleotide sequences have homology, similarity, or identity may be confirmed through Southern hybridization experiments under appropriate hybridization conditions. The appropriate hybridization conditions can be determined by methods well known to those skilled in the art (such as J. Sambrook et al., Molecular Cloning, A Laboratory Manual; F.M. Ausubel et al., Current Protocols in Molecular Biology, John Wiley & Sons, Inc., New York), but are not limited thereto. For example, homologous or identical polynucleotide sequences can typically hybridize under stringent conditions along the whole sequence or at least about 50%, 60%, 70%, 80%, or 90% of the full length.

As used herein, the term "stringent conditions" refers to conditions that enable specific hybridization between polynucleotides. These conditions are specifically described in the literature (see Sambrook *et al.*, supra, 9.50-9.51, 11.7-11.8). For example, the stringent conditions may comprise conditions under which polynucleotides having high homology or identity, *i.e.*, polynucleotides having at least 60%, at least 70%, at least 75%, at least 80%, at least 85%, at least 90%, at least 95%, at least 96%, at least 97%, at least 98%, or at least 99% homology or identity hybridize with each other, while polynucleotides having homology or identity lower than the above homology or identity do not hybridize with each other, or may comprise ordinary washing conditions of Southern hybridization, *i.e.*, washing once, specifically twice or three times, at a salt concentration and temperature corresponding to 60°C, 1XSSC, 0.1% SDS, specifically 60°C, 0.1XSSC, 0.1% SDS, and more specifically 68°C, 0.1XSSC, 0.1% SDS.

The hybridization may occur between nucleotides having complementary base sequences, but the hybridized polynucleotides may comprise some base mismatches depending on the stringency of the hybridization. The term "complementary" is used to describe a relationship between nucleotide bases that can hybridize with each other. For example, regarding DNA, adenosine is complementary to thymine, and cytosine is complementary to guanine. Therefore, the polynucleotide of the present disclosure may comprise an isolated nucleic acid fragment complementary to the entire sequence, as well as a base sequence substantially similar thereto.

Specifically, polynucleotides having homology or identity to the polynucleotide of the present disclosure may be detected through hybridization at a Tₘ value of 55°C. Further, the Tₘ value may be 60°C, 63°C, or 65°C, but is not limited thereto, and may be appropriately adjusted by those skilled in the art.

The appropriate stringency for hybridizing the polynucleotides depends on the length and degree of complementarity of the polynucleotides, and the variables are well known in the art (*e.g.*, Sambrook *et al.*, supra).

### Vectors, Transformation

As used herein, the term "vector" refers to a DNA construct for delivering a desired polynucleotide into a suitable host or host cell.

In one example, the vector may comprise a base sequence of a polynucleotide encoding a desired polypeptide that is operably linked to a suitable expression regulatory region (or expression regulatory sequence) such that the desired polypeptide can be expressed in a suitable host. The expression regulatory sequence may comprise a promoter capable of initiating transcription, any operator sequence for regulating the transcription, a sequence encoding a suitable mRNA ribosome-binding site, and a sequence for regulating termination of transcription and translation. The vector, after being transformed into a suitable host cell (microorganism), may replicate or function independently of the host genome, or may be integrated into the genome itself to replicate or function.

Additionally, in one example, the vector of the present disclosure may comprise a sequence for inserting a desired polynucleotide into a chromosome. The insertion of a polynucleotide into a chromosome using the vector may be performed by any method known in the art, for example, homologous recombination, but is not limited thereto.

The vector used in the present disclosure is not particularly limited, and any vector known in the art may be used. Examples of commonly used vectors comprise plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc.*, may be used; and as a plasmid vector, those based on pDZ, pDC, pBR, pUC, pBluescriptII, pGEM, pTZ, pCL, pET, *etc.*, may be used. In one example, pDZ, pDC, pDCM2, pACYC177, pACYC184, pCL, pECCG117, pUC19, pBR322, pMW118, pCC1BAC vectors, *etc.*, may be used.

The vector may further comprise a selection marker for confirming the transformation into a host cell, or further, the insertion into a chromosome of the host cell. The selection marker is used to select cells transformed with the vector or to confirm the insertion of a desired polynucleotide into a chromosome; markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a distinct phenotype, allowing for the selection of transformed cells.

As used herein, the term "transformation" refers to the introduction of a desired polynucleotide or a vector comprising the same into a host cell (microorganism), thereby altering the genetic traits of the host cell (microorganism). The transformed polynucleotide may be inserted into the chromosome of a host cell (microorganism) or located outside the chromosome. Further, the polynucleotide may comprise DNA or RNA. The polynucleotide may be introduced in a suitable form depending on the purpose of introduction. For example, a polynucleotide for expression of a desired polypeptide may be introduced into a host cell (microorganism) in the form of an expression cassette, which is a gene construct comprising all elements required for self-expression. The expression cassette may typically comprise a promoter that is operably linked to the coding sequence of the desired polypeptide, a transcription termination signal, a ribosome binding site, and a translation termination signal. The expression cassette may be in the form of a self-replicating expression vector. Further, the polynucleotide may be introduced into a host cell (microorganism) as-is and may be operably linked to a sequence required for expression in the host cell (microorganism), but is not limited thereto.

As used herein, the term "operably linked" refers to a configuration in which a regulatory sequence is positioned at an appropriate position to regulate the expression of a coding sequence. Thus, the term "operably linked" comprises attaching or linking a regulatory region of a functional domain having a known or desired activity such as a promoter, a stop codon, a signal sequence, or an enhancer region, with a target (gene or polypeptide) so as to regulate the expression, secretion, or function of the target in accordance with the known or desired activity. For example, it may mean that a polynucleotide sequence encoding a polypeptide is functionally linked to a promoter sequence that initiates and mediates the transcription of the polynucleotide.

As used herein, the term "expression" comprises any step involved in the production of a polypeptide, for example, transcription, post-transcriptional modification, translation, post-translational modification, secretion, and the like, but is not limited thereto.

As used herein, the term "expression vector" refers to a linear or circular nucleic acid molecule comprising a desired polynucleotide sequence and an operably linked regulatory sequence for expression thereof. For example, it may comprise a base sequence of a polynucleotide encoding a desired polypeptide that is operably linked to a suitable expression regulatory region (or expression regulatory sequence) such that the desired polypeptide can be expressed in a suitable host.

As used herein, the term "regulatory sequence" refers to a polynucleotide sequence necessary for regulating expression of a desired polynucleotide sequence. Each regulatory sequence may be native (derived from the same origin) or foreign (derived from a different gene) to the coding sequence, a variant sequence thereof, or another artificial sequence. Examples of regulatory sequences may include a leader sequence, a polyadenylation sequence, a pro-peptide sequence, a promoter, a signal peptide sequence, an operator sequence, a sequence encoding a ribosome-binding domain, and a sequence regulating termination of transcription and translation. The minimum unit of a regulatory sequence may comprise a promoter and a sequence for terminating transcription and translation.

As used herein, the term "genetic recombination" refers to a natural or artificial process in which an element constituting a gene, such as DNA or RNA, is rearranged into an order different from the original sequence during disassembly and reassembly processes.

As used herein, the term "recombinant gene" refers to a gene having a new genomic configuration that results from genetic recombination, such as chemical synthesis or genetic engineering techniques. In the present disclosure, the terms "recombinant gene", "recombinant DNA", and "recombinant polynucleotide" may be used interchangeably. In one example, the recombinant gene may comprise an artificial combination of nucleic acid fragments such as regulatory sequences that are not naturally found together.

As used herein, the term "recombinant protein" refers to a protein produced as a result of genetic recombination.

### Microorganisms

As used herein, the term "microorganism (or strain)" comprises both wild-type microorganisms and prokaryotic or eukaryotic microorganisms that have undergone genetic modification naturally or artificially. It may be a microorganism in which a specific mechanism is weakened or enhanced due to the insertion of a foreign gene or enhancement or inactivation of activity of an endogenous gene, and may be a microorganism comprising a genetic modification for the production of a desired polypeptide, protein, or product. In the present disclosure, the terms "microorganism", "strain", "host", and "host cell" may be used interchangeably.

As used herein, the term "recombinant microorganism" refers to a microorganism that has been genetically modified to exhibit a different genotype and/or phenotype compared to a naturally occurring microorganism (*e.g.*, when genetic modification affects how the nucleic acid sequence is encoded in the microorganism), and may comprise all progeny or potential progeny of the microorganism. In the present disclosure, the terms "recombinant microorganism", "genetically modified microorganism", "recombinant host cell", "recombinant cell", and "recombinant strain" may be used interchangeably. The recombinant microorganism may, for example, express a gene that is not found in its natural (non-recombinant) form, not express a gene that is expressed in its natural form, or express a native gene in a manner different from that in which it is expressed in its natural form.

As used herein, the term "non-modified microorganism (strain)" does not exclude a microorganism (strain) comprising mutations that may occur naturally, and may refer to a wild-type microorganism (strain) or a natural microorganism (strain) as-is, or a microorganism (strain) before a trait is altered by genetic variation due to natural or artificial factors. In the present disclosure, the term "non-modified microorganism (strain)" may be used interchangeably with "microorganism (strain) before modification", "non-mutated microorganism (strain)", "parent microorganism", "parent strain", "wild-type microorganism (strain)", "reference microorganism (strain)", or "standard organism (strain)".

### Culturing

As used herein, the term "culturing" refers to growing a microorganism under suitably controlled environmental conditions. The culturing process may be performed in a suitable medium known in the art under suitable culturing conditions known in the art. Such a culturing process may be easily adjusted and used by those skilled in the art according to the selected microorganism. Specifically, the culturing may be batch culturing, continuous culturing, and/or fed-batch culturing, but is not limited thereto.

As used herein, the term "medium" refers to a mixed substance containing nutrients required for culturing a microorganism as main components, and the medium supplies nutrients, growth factors, *etc.*, including water, which are indispensable for survival and development. Specifically, any medium and culture conditions may be used for culturing the microorganism of the present disclosure without particular limitation, as long as the medium is used for the common culture of microorganisms. For example, the microorganism of the present disclosure may be cultured in a common medium containing appropriate carbon sources, nitrogen sources, phosphorus sources, inorganic compounds, amino acids, and/or vitamins, *etc.*, while controlling the temperature, pH, *etc.* under aerobic conditions.

In the present disclosure, the carbon sources comprise carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.*, molasses converted into reducing sugars) may be used, and other various carbon sources in appropriate amounts may be used without limitation. These carbon sources may be used alone or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids, such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more thereof, but are not limited thereto.

As the phosphorus sources, monopotassium phosphate, dipotassium phosphate, or sodium-containing salts corresponding thereto may be used. As for inorganic compounds, sodium chloride, calcium chloride, iron chloride, magnesium sulfate, iron sulfate, manganese sulfate, calcium carbonate, *etc.*, may be used, and in addition, amino acids, vitamins, and/or suitable precursors may be comprised. These components or precursors may be added to the medium in a batchwise or continuous manner. However, the medium is not limited thereto.

During the culturing of the microorganism of the present disclosure, the pH of the medium may be adjusted by adding a compound such as ammonium hydroxide, potassium hydroxide, ammonia, phosphoric acid, or sulfuric acid to the medium in an appropriate manner. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas may be injected, or nitrogen, hydrogen, or carbon dioxide gas may be injected. However, the culturing conditions are not limited thereto.

In the culturing of the present disclosure, the culturing temperature may be maintained at 20°C to 45°C, specifically 25°C to 40°C, and the culturing may be performed for about 10 to 160 hours, but the culturing conditions are not limited thereto.

As used herein, the term "culture" refers to a culture solution, a concentrated culture solution, a dried product of the culture solution, a culture filtrate, a concentrated culture filtrate, or a dried product of the culture filtrate obtained by culturing a specific microorganism in a culture medium. The culture solution refers to a solution comprising a specific microorganism, whereas the culture filtrate refers to a solution that substantially does not contain the specific microorganism (in particular, "substantially" means that the specific microorganism separated by filtration or the like is excluded, but does not mean that the microorganism is completely absent from the filtrate). The culture is not limited in its form, and, in one example, may be in the form of a liquid, an emulsion, or a solid.

### Detailed Description of the Present Disclosure

Hereinafter, the specific embodiments of the present disclosure are described in more detail.

An aspect of the present disclosure provides a polypeptide comprising: the amino acid sequence of SEQ ID NO: 5 or 7; and an amino acid sequence of a *Serratia marcescens*-derived nuclease.

In the present disclosure, the amino acid sequence of SEQ ID NO: 5 or 7 refers to a signal peptide sequence. In particular, the signal peptide is a type of target peptide, and refers to a region consisting of 15 to 30 amino acids that is located at the N-terminus of a secretory protein or a membrane protein and serves as a signal when the protein passes through a membrane.

In the present disclosure, the signal peptide may be a peptide derived from a microorganism of the genus *Bacillus*, specifically may be a peptide derived from *Bacillus subtilis*, and more specifically may be a yqxI or yoaW signal peptide derived from *Bacillus subtilis*. In particular, the yqxI signal peptide has the amino acid sequence of SEQ ID NO: 5, consisting of 28 amino acids, and the yoaW signal peptide has the amino acid sequence of SEQ ID NO: 7, consisting of 24 amino acids.

In the present disclosure, the sequence of a polynucleotide encoding the amino acid sequence of SEQ ID NO: 5 or 7 can be obtained based on codon information known in the art. In one example, the polynucleotide encoding the amino acid sequence of SEQ ID NO: 5 may have, comprise, consist of, or essentially consist of the sequence of SEQ ID NO: 6 or a base sequence having at least 60%, at least 70%, at least 80%, at least 85%, at least 90%, at least 95%, at least 96%, at least 97%, at least 98%, or at least 99% homology or identity to the sequence of SEQ ID NO: 6, but is not limited thereto, and any polynucleotide may be included without limitation, as long as it is capable of functioning as the signal peptide of the present disclosure in a manner identical or corresponding to the nucleic acid molecule consisting of the polynucleotide. Additionally, the polynucleotide encoding the amino acid sequence of SEQ ID NO: 7 may have, comprise, consist of, or essentially consist of the sequence of SEQ ID NO: 8 or a base sequence having at least 60%, at least 70%, at least 80%, at least 85%, at least 90%, at least 95%, at least 96%, at least 97%, at least 98%, or at least 99% homology or identity to the sequence of SEQ ID NO: 6, but is not limited thereto, and any polynucleotide may be included without limitation, as long as it is capable of functioning as the signal peptide of the present disclosure in a manner identical or corresponding to the nucleic acid molecule consisting of the polynucleotide. In particular, "homology" or "identity" is as described above.

In the present disclosure, the amino acid sequence of SEQ ID NO: 5 or 7 may be directly fused to the N-terminus of the amino acid sequence of the nuclease, or be operably linked to the amino acid sequence of the nuclease via a linker.

"*Serratia marcescens*" of the present disclosure refers to a Gram-negative bacterium belonging to the family *Enterobacteriaceae*, which is known to be widely distributed in water, soil, foods, and the like.

As used herein, the term "nuclease" refers to an enzyme that cleaves phosphodiester bonds in nucleic acids, is known to perform an important role in replication, restoration, recombination, and degradation of DNA and RNA in living organisms, and comprises deoxyribonucleases (DNases) and ribonucleases (RNases). In the present disclosure, the nuclease may be a *Serratia marcescens*-derived nuclease, and the *Serratia marcescens*-derived nuclease is known to possess a non-specific feature of cleaving various forms of DNA and RNA, but is not limited thereto.

In the present disclosure, the amino acid sequence of the *Serratia marcescens-*derived nuclease and a polynucleotide sequence encoding the same can be obtained from a publicly known database. Examples of the database include GenBank of NCBI, but are not limited thereto. In the present disclosure, the *Serratia marcescens*-derived nuclease may have, comprise, consist of, or essentially consist of the amino acid sequence of SEQ ID NO: 1.

In the present disclosure, the *Serratia marcescens*-derived nuclease may comprise an amino acid sequence having at least 80%, at least 85%, at least 90%, at least 95%, at least 96%, at least 97%, at least 98%, at least 99%, at least 99.5%, at least 99.7%, or at least 99.9% homology or identity to the amino acid sequence of SEQ ID NO: 1. Additionally, it is apparent that any protein having an amino acid sequence, in which part of the sequence is deleted, modified, substituted, conservatively substituted, or added, may also fall within the scope of the present disclosure, as long as the amino acid sequence has such a homology or identity and exhibits an equivalent efficacy to that of the protein comprising the amino acid sequence of SEQ ID NO: 1.

In the present disclosure, the sequence of a polynucleotide encoding the amino acid sequence of SEQ ID NO: 1 or an amino acid sequence having at least 80% homology or identity thereto can be obtained based on codon information known in the art. In the present disclosure, the *Serratia marcescens*-derived nuclease may be encoded by a polynucleotide having, comprising, consisting of, or essentially consisting of a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 2, but is not limited thereto. The base sequence of SEQ ID NO: 2 can be obtained from a publicly known database. Examples of the database include GenBank of NCBI, but are not limited thereto.

The polynucleotide of the present disclosure may undergo various modifications in the coding region within the scope that does not change the amino acid sequence of the nuclease of the present disclosure, taking into consideration codon degeneracy or codons preferred in an organism in which the nuclease of the present disclosure is to be expressed. Accordingly, it is apparent that a polynucleotide that can be translated, by codon degeneracy, into a polypeptide consisting of an amino acid sequence of the nuclease of the present disclosure or a polypeptide having a homology or identity thereto may also be comprised in the polynucleotide of the present disclosure.

Additionally, the polynucleotide of the present disclosure may comprise, without limitation, a probe that can be prepared from a known gene sequence, for example, any sequence encoding the nuclease of the present disclosure by hybridizing with a sequence complementary to all or part of the polynucleotide of the present disclosure under stringent conditions.

The polypeptide of the present disclosure may further comprise the amino acid sequence of SEQ ID NO: 11, in addition to the amino acid sequence of SEQ ID NO: 5 or 7 and the amino acid sequence of a *Serratia marcescens*-derived nuclease.

In the present disclosure, the amino acid sequence of SEQ ID NO: 11 refers to a pro-peptide sequence. In particular, the pro-peptide refers to a protein precursor that is an inactive protein capable of being converted into an active form through post-translational modification, and is also used interchangeably with a pre-pro-peptide. Multiple pro-peptides are known to be synthesized together with a signal peptide for N-terminal secretion.

In the present disclosure, the pro-peptide may be a peptide derived from a microorganism of the genus *Bacillus*, specifically may be a peptide derived from *Bacillus subtilis*, and more specifically may be an amyE pro-peptide derived from *Bacillus subtilis*. In particular, the amyE pro-peptide has the amino acid sequence of SEQ ID NO: 11, consisting of 8 amino acids.

In the present disclosure, the sequence of a polynucleotide encoding the amino acid sequence of SEQ ID NO: 11 can be obtained based on codon information known in the art. In one example, the polynucleotide may have, comprise, consist of, or essentially consist of the sequence of SEQ ID NO: 12 or a base sequence having at least 60%, at least 70%, at least 80%, at least 85%, at least 90%, at least 95%, at least 96%, at least 97%, at least 98%, or at least 99% homology or identity to the sequence of SEQ ID NO: 12, but is not limited thereto, and any nucleic acid molecule may be comprised without limitation, as long as it is capable of functioning as the pro-peptide of the present disclosure in a manner identical or corresponding to the nucleic acid molecule consisting of the polynucleotide. In particular, "homology" or "identity" is as described above.

In the present disclosure, the amino acid sequence of SEQ ID NO: 11 may be directly fused to the C terminus of the amino acid sequence of SEQ ID NO: 5 or 7 and the N-terminus of the amino acid sequence of the nuclease, and each of the amino acid sequence of SEQ ID NO: 5 or 7, the amino acid sequence of SEQ ID NO: 11, and the amino acid sequence of the nuclease may be operably linked to each other.

Another aspect of the present disclosure provides a polynucleotide encoding a polypeptide comprising: an amino acid sequence of SEQ ID NO: 5 or 7; and an amino acid sequence of a *Serratia marcescens*-derived nuclease.

The "amino acid sequence of SEQ ID NO: 5 or 7" and the "amino acid sequence of a *Serratia marcescens*-derived nuclease" of the present disclosure are as described above.

The polynucleotide encoding a polypeptide comprising: the amino acid sequence of SEQ ID NO: 5 or 7; and the amino acid sequence of a *Serratia marcescens*-derived nuclease of the present disclosure can be obtained based on codon information known in the art. The polynucleotide may comprise the base sequence of a polynucleotide encoding a polypeptide in which the amino acid sequence of SEQ ID NO: 5 or 7 and the amino acid sequence of a *Serratia marcescens*-derived nuclease are operably linked, and may have, comprise, consist of, or essentially consist of a base sequence having at least 60%, at least 70%, at least 80%, at least 85%, at least 90%, at least 95%, at least 96%, at least 97%, at least 98%, or at least 99% homology or identity to the base sequence, but is not limited thereto.

Still another aspect of the present disclosure provides an expression vector comprising a polynucleotide encoding the polypeptide of the present disclosure.

The term "expression vector" of the present disclosure is as described above.

Still another aspect of the present disclosure provides a microorganism comprising the polypeptide of the present disclosure or a polynucleotide encoding the same.

In the present disclosure, the term "microorganism" is as described above. In the present disclosure, the microorganism comprising the polypeptide or a polynucleotide encoding the same may comprise any microorganism, as long as the microorganism is capable of expressing a nuclease by comprising the polypeptide of the present disclosure or a polynucleotide encoding the same. Specifically, the microorganism of the present disclosure may be a microorganism of the genus *Bacillus*, and more specifically *Bacillus subtilis*, but is not limited thereto. Additionally, the microorganism may be a recombinant microorganism transformed with a vector comprising a polynucleotide encoding the polypeptide of the present disclosure, through which a *Serratia marcescens*-derived nuclease may be expressed or overexpressed in the microorganism. In particular, the method for transforming the microorganism with the vector includes any method of introducing a nucleic acid into a cell and may be performed by selecting a suitable standard technique as known in the art in accordance with the host cell.

Still another aspect of the present disclosure provides a method for producing a *Serratia marcescens*-derived nuclease, comprising: culturing a microorganism, comprising the polypeptide of the present disclosure or a polynucleotide encoding the same, in a medium; and recovering a nuclease from the culture.

The term "culturing" of the present disclosure is as described above.

In the method for producing a *Serratia marcescens*-derived nuclease of the present disclosure, the "recovering" refers to obtaining a desired product from the microorganism or the culture, and methods well known in the art, such as centrifugation, filtration, anion-exchange chromatography, crystallization, and HPLC, may be used, but are not limited thereto. The recovering may comprise a purification process, and those skilled in the art can select and employ any of various purification processes known in the art as necessary.

Still another aspect of the present disclosure provides a composition for producing a *Serratia marcescens*-derived nuclease, comprising a signal peptide having the amino acid sequence of SEQ ID NO: 5 or 7.

In the composition of the present disclosure, the amino acid sequence of SEQ ID NO: 5 or 7, the signal peptide, and the *Serratia marcescens*-derived nuclease are as described above.

The composition for producing a *Serratia marcescens*-derived nuclease of the present disclosure may further comprise a peptide having the amino acid sequence of SEQ ID NO: 11, and the amino acid sequence of SEQ ID NO: 11 is as described above.

Still another aspect of the present disclosure provides the use of a signal peptide having an amino acid sequence of SEQ ID NO: 5 or 7 for producing a nuclease.

### [Mode for Carrying Out the Invention]

Hereinafter, the present disclosure is described in detail by way of Examples. The Examples are provided to specifically explain the present disclosure, and the scope of the present disclosure is not limited by the Examples.

### Example 1. Screening of signal peptides and construction of Serratia marcescens-derived nuclease producing strains

A signal peptide screening was performed using a *Bacillus subtilis* Secretory Protein Expression System (Takara). A nuclease (SEQ ID NO: 1) was synthesized by Cosmo Genetech after codon optimization for *B. subtilis* based on a *Serratia marcescens-*derived nucA sequence. The synthesized sequence was amplified by PCR using the primers of SEQ ID NOS: 3 and 4, and the PCR product was ligated into a pBE-S vector treated with NdeI and EcoRI using an In-Fusion HD cloning kit (Clontech). The resulting construct was transformed into DH5α to obtain colonies. Plasmids were purified from the obtained colonies to prepare a nuclease expression vector (pBE-SPaprE_nucA).

In order to construct a library of nuclease-producing strains for each signal peptide, an SP DNA mixture (Takara) encoding 173 types of *B. subtilis*-derived secretion signal peptides and pBE-SPaprE-nucA treated with MluI and EagI were ligated using an In-Fusion^{®} HD cloning kit (Clontech). The ligation products were transformed into *Bacillus subtilis* (BGSC 1S145) using a natural competency method (Bron, 1996), thereby securing approximately 400 libraries.

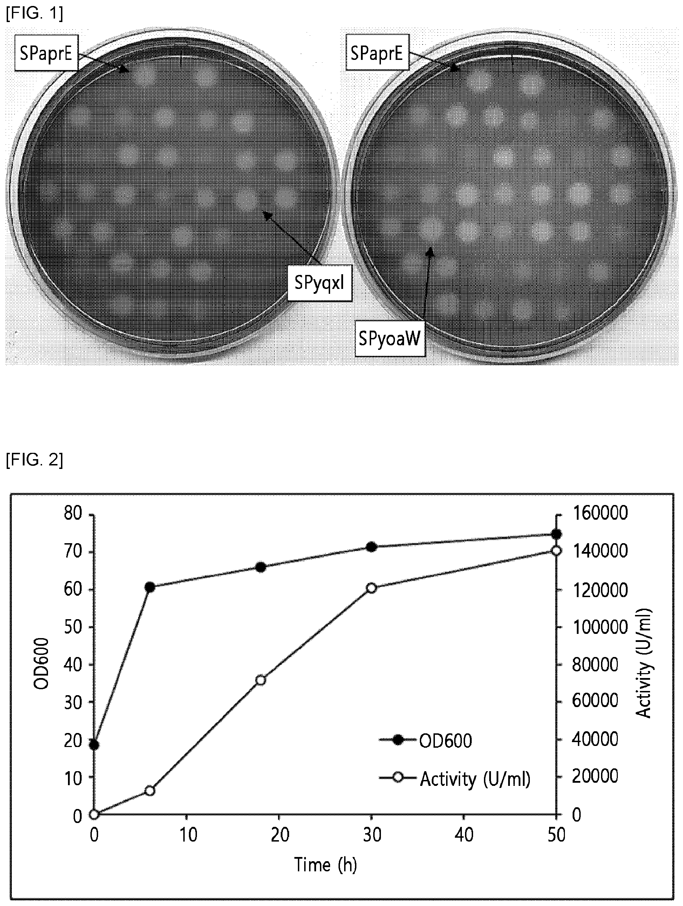

For screening of the constructed nuclease-producing strains, the generated colonies were inoculated into 96 deep-well plates containing 300 µL of LB medium (10 g/L tryptone, 5 g/L yeast extract, 10 g/L NaCl, and 50 µg/mL kanamycin) and incubated at 37°C and 800 rpm for 24 hours. Thereafter, 3 µL of the supernatant was spotted onto DNase agar with methyl green (KisanBio Co., Ltd.) plates, incubated at 37°C for 2 hours, and the generated halo size was confirmed (FIG. 1).

Based on the above results, superior signal peptides were selected, and it was confirmed through sequencing that yqxI (SEQ ID NO: 5) and yoaW (SEQ ID NO: 7) signal peptide sequences were fused.

In addition to the selected strains, a strain in which a signal peptide of the *B. subtilis* amyE gene was fused was additionally constructed. Specifically, PCR was performed using *B. subtilis* 168 gDNA as a template and the primers of SEQ ID NOS: 9 and 10, followed by ligation with pBE-SPaprE-nucA treated with MluI and EagI using an In-Fusion^{®} HD cloning kit (Clontech). The resulting construct was transformed into *Bacillus subtilis* (BGSC 1S145) to prepare a strain BNUC4 (SPamyE).

### Example 2. Comparative evaluation of nuclease expression for each signal peptide

Through flask evaluation of each strain BNUC1 (SPaprE), BNUC2 (SPyqxI), BNUC3 (SPyoaW), and BNUC4 (SPamyE) selected and constructed through the above process, the nuclease expression level for each signal peptide was compared. Each strain was incubated overnight at 37°C in LB medium, and then inoculated at 1% into 25 mL of a titer medium (10 g/L glucose, 15 g/L CSL, 20 g/L yeast extract, 2 g/L MgCl₂, 2 g/L NaSO₄, 2 g/L (NH₄)₂SO₄, and 1.5 g/L K₂HPO₄). After incubation at 37°C and 200 rpm for 18 hours, nuclease activity in the supernatant was measured. The method for evaluating nuclease activity is as follows.

### (1) Materials

1 M Tris-HCl (Biosesang), MgCl₂ (Daejung Chemicals), albumin from bovine serum (BSA, Sigma-Aldrich), salmon sperm DNA (Invitrogen), and perchloric acid 70% (Sigma-Aldrich)

### (2) Buffers

1) Reagent A (1mM MgCl2, 0.1 mg/ml BSA in 50mM Tris-HCl, pH 8.0)
2) Reagent B (0.1mg/ml Salmon sperm DNA in reagent A)
3) 4% Perchloric acid solution

### (3) Procedure

A sample appropriately diluted with Reagent A (25 µL) or 25 µL of Reagent A as a blank was mixed with 500 µL of Reagent B, followed by reaction at 37°C for 30 minutes. Thereafter, 525 µL of 4% perchloric acid solution was added to terminate the reaction, and the mixture was placed on ice for 30 minutes. The mixture was then centrifuged at 14,000 rpm for 6 minutes, and the supernatant was separated to measure absorbance at 260 nm. In particular, one unit was defined as the amount of enzyme required to produce a change in absorbance of 1.0 at 260 nm for 30 minutes under optimal conditions in the presence of excess substrate.

As a result, as shown in Table 1 below, it was confirmed that nuclease activity was significantly superior when the yqxI or yoaW signal peptide was used, as compared to when the aprE or amyE signal peptide was used.

**[Table 1]**

| Strain | Description | Nuclease Activity (Unit/mL) |
|---|---|---|
| BNUC1 | BGSC 1S145 pBE-SPaprE_nucA | 8946 |
| BNUC2 | BGSC 1S145 pBE-SPyqxI_nucA | 15864 |
| BNUC3 | BGSC 1S145 pBE-SPyoaW_nucA | 16238 |
| BNUC4 | BGSC 1S145 pBE-SPamyE_nucA | 3790 |

### Example 3. Confirmation of the effect of increased nuclease expression upon fusion of a Pro-amyE peptide to a signal peptide

In order to confirm the effect of an amyE pro-peptide on nuclease secretion efficiency, eight amino acids (SEQ ID NO: 11) were additionally fused to the C-terminus of each of the yqxI or yoaW signal peptide sequences. Specifically, PCR products were obtained using the vectors pBE-SPyqxI_nucA or pBE-SPyoaW_nucA constructed in Example 1 as templates and the primers of SEQ ID NOS: 13, 14, 15, and 16, and the PCR products were respectively ligated using an In-Fusion HD cloning kit (Clontech) to prepare nuclease expression vectors in which Pro-amyE was fused. The resulting constructs were transformed into *Bacillus subtilis* (BGSC 1S145), and evaluation was performed.

The strains were incubated overnight at 37°C in LB medium and then inoculated at 1% into 25 mL of a titer medium (10 g/L glucose, 15 g/L CSL, 20 g/L yeast extract, 2 g/L MgCl₂, 2 g/L NaSO₄, 2.68 g/L (NH₄)₂SO₄, 1.5 g/L K₂HPO₄, and 50 µg/mL kanamycin). After incubation at 37°C and 200 rpm for 18 hours, nuclease activity in the supernatant was measured.

As a result, as shown in Table 2 below, it was confirmed that, for both signal peptides yqxI and yoaW, fusion with Pro-amyE exhibited a synergistic effect in which activity was further increased by at least 20%.

**[Table 2]**

| Strain | Description | Nuclease Activity (Unit/mL) |
|---|---|---|
| BNUC1 | BGSC 1S145 pBE-SPaprE_nucA | 9837 |
| BNUC2 | BGSC 1S145 pBE-SPyqxI_nucA | 16987 |
| BNUC3 | BGSC 1S145 pBE-SPyoaW_nucA | 18125 |
| BNUC5 | BGSC 1S145 pBE-SPyqxI_Pro-amyE_nucA | 20856 |
| BNUC6 | BGSC 1S145 pBE-SPyoaW_Pro-amyE_nucA | 22385 |

### Example 4. Validation of nuclease activity in a 5L fermenter culture .

Nuclease expression levels in a strain were confirmed using a 5 L fermenter according to the following method. A BNUC6 strain was seed-cultured in 300 mL of LB medium containing 50 µg/mL kanamycin, and then inoculated into 1.8 L of a fermentation medium (40 g/L glucose, 50 g/L CSL, 20 g/L yeast extract, 2 g/L MgSO₄, 4 g/L (NH₄)₂SO₄, 2 g/L KH₂PO₄, 4 g/L CaCl₂, and 50 µg/mL kanamycin). Air was supplied at a pH of 6.7, 800 rpm, and 1 vvm. Culturing was performed by maintaining the temperature at 37°C and switching to 32.5°C after 6 hours of culturing, while feeding a feed medium (250 g/L glucose, 300 g/L CSL, and 20 g/L yeast extract) at a rate of 12 mL/h.

After the culturing, sampling was performed at 6 hours, 18 hours, 30 hours, and 50 hours, respectively, and nuclease expression activity in the supernatant was measured. As a result, as shown in FIG. 2, it was confirmed that the strain ultimately exhibited a nuclease expression activity of at least 140,000 U/mL.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as comprising the meaning and scope of the appended claims rather than the detailed description disclosed above, and all changes or variations derived from the equivalent concepts fall within the scope of the present disclosure.

**[Table 3]**

| [Sequence Listing] | | |
|---|---|---|
| SEQ ID NO | Name | Sequence |
| 1 | *Serratia marcescen s* derived Nuclease AA | DTLESIDNCAVGCPTGGSSNVSIVRHAYTLNNNSTTKFANWVA YHITKDTPASGKTRNWKTDPALNPADTLAPADYTGANAALKVD RGHQAPLASLAGVSDWESLNYLSNITPQKSDLNQGAWARLED QERKLIDRADISSVYTVTGPLYERDMGKLPGTQKAHTIPSAYW KVIFINNSPAVNHYAAFLFDQNTPKGADFCQFRVTVDEIEKRTG LIIWAGLPDDVQASLKSKPGVLPELMGCKN |
| 2 | *Serratia marcescen s* derived Nuclease NT | GATACACTGGAATCAATTGATAATTGCGCAGTTGGCTGCCCG ACGGGCGGCAGCTCAAATGTTTCAATTGTGAGACACGCATA CACATTGAATAATAATTCAACAACGAAGTTTGCAAATTGGGTT GCATATCATATTACAAAAGATACACCGGCAAGCGGCAAAACA AGAAATTGGAAAACAGATCCGGCACTGAATCCGGCAGATAC ACTGGCACCGGCAGATTATACGGGCGCAAATGCAGCACTGA AAGTTGATAGAGGCCATCAAGCACCGCTGGCATCACTGGCG GGCGTTTCAGATTGGGAATCACTGAATTATCTGTCAAATATTA CACCGCAAAAATCAGATCTGAATCAAGGCGCATGGGCAAGA |
| | | CTGGAAGATCAAGAAAGAAAACTGATTGATAGAGCAGATATT TCATCAGTTTATACAGTTACGGGCCCGCTGTATGAAAGAGAT ATGGGCAAACTGCCGGGCACACAAAAAGCACATACAATTCC GTCAGCATATTGGAAAGTTATTTTTATTAATAATTCACCGGCA GTTAATCATTATGCAGCATTTCTGTTTGATCAAAATACACCGA AAGGCGCAGATTTTTGCCAATTTAGAGTTACAGTTGATGAAAT TGAAAAAAGAACGGGCCTGATTATTTGGGCGGGCCTGCCGG ATGATGTTCAAGCATCACTGAAATCAAAACCGGGCGTTCTGC CGGAACTGATGGGCTGCAAAAATTGA |
| 3 | nucA F Primer NT | GCGGCCGGTGCACAT GATACACTGGAATCAATTGATAATTGCG |
| 4 | nucA R Primer NT | TGCAGGTCGACAAGCTT TCAATTTTTGCAGCCCATCAGTTC |
| 5 | yqxI Signal peptide AA | MFKKLLLATSALTFSLSLVLPLDGHAKA |
| 6 | yqxI Signal peptide NT | ATGTTTAAGAAATTACTTTTAGCAACATCTGCATTAACATTCTC TTTATCATTAGTTCTTCCGTTGGATGGACATGCCAAAGCT |
| 7 | yoaW Signal peptide AA | MKKMLMLAFTFLLALTIHVGEASA |
| 8 | yoaW Signal peptide NT | ATGAAAAAGATGTTGATGTTAGCTTTTACATTTCTTTTGGCTTT GACTATCCATGTAGGGGAAGCTTCGGCT |
| 9 | SPamyE F primer NT | AGGAGAGGGACGCGT ATGTTTGCAAAACGATTCAAAACC |
| 10 | SPamyE R primer NT | TGATTCCAGTGTATC AGCACTCGCAGCCGCCGG |
| 11 | amyE Pro-peptide AA | ETANKSNE |
| 12 | amyE Pro-peptide NT | GAAACGGCGAACAAATCGAATGAG |
| 13 | SPyqxI_P RO-amyE fusion F primer NT | GAAACGGCGAACAAATCGAATGAG GATACACTGGAATCAATTGATAATTGCG |
| 14 | SPyqxI_P RO-amyE fusion R primer NT | CTCATTCGATTTGTTCGCCGTTTC AGCTTTGGCATGTCCATCCAAC |
| 15 | SPyoaW_ PRO-amyE fusion F primer NT | GAAACGGCGAACAAATCGAATGAG GATACACTGGAATCAATTGATAATTGCG |
| 16 | SPyoaW_ PRO-amyE fusion R primer NT | CTCATTCGATTTGTTCGCCGTTTC GGCCGAAGCTTCCCCTACAT |

## Claims

1. A polypeptide comprising: an amino acid sequence of SEQ ID NO: 5 or 7; and an amino acid sequence of a *Serratia marcescens*-derived nuclease.

2. The polypeptide according to claim 1, wherein the amino acid sequence of the *Serratia marcescens*-derived nuclease has at least 80% homology with the amino acid sequence of SEQ ID NO: 1.

3. The polypeptide according to claim 1, wherein the amino acid sequence of SEQ ID NO: 5 or 7 is fused to the N-terminus of the amino acid sequence of the nuclease.

4. The polypeptide according to claim 1, wherein the polypeptide further comprises an amino acid sequence of SEQ ID NO: 11, and wherein the amino acid sequence of SEQ ID NO: 11 is fused to the C-terminus of the amino acid sequence of SEQ ID NO: 5 or 7 and to the N-terminus of the amino acid sequence of the nuclease.

5. A polynucleotide encoding the polypeptide of any one of claims 1 to 4.

6. A microorganism comprising the polypeptide of any one of claims 1 to 4 or a polynucleotide encoding the same.

7. The microorganism of claim 6, wherein the microorganism is a microorganism of the genus *Bacillus*.

8. The microorganism of claim 6, wherein the microorganism is *Bacillus subtilis*.

9. A method for producing a *Serratia marcescens*-derived nuclease, comprising: culturing a microorganism, comprising the polypeptide of any one of claims 1 to 4 or a polynucleotide encoding the same, in a medium; and recovering a nuclease from the culture.

10. A composition for producing a *Serratia marcescens*-derived nuclease, comprising a signal peptide having an amino acid sequence of SEQ ID NO: 5 or 7.

11. The composition according to claim 10, further comprising a peptide having an amino acid sequence of SEQ ID NO: 11.

12. Use of a signal peptide having an amino acid sequence of SEQ ID NO: 5 or 7 for producing a nuclease.
